# EUROPEAN PATENT SPECIFICATION

(11) **EP 1 286 680 B1**
(45) Date of publication and mention of the grant of the patent: **26.11.2008**
(21) Application number: 00946795.2
(22) Date of filing: 10.07.2000
(51) Int. Cl.: A61K 36/03, A61P 37/02

(54) **SEAWEED SUPPLEMENT DIET FOR ENHANCING IMMUNE RESPONSE IN MAMMALS**
ALGEN ALS NAHRUNGSERGÄNZUNG ZUR STEIGERUNG DER IMMUNANTWORT IN SÄUGETIEREN
REGIME A COMPLEMENT A BASE D'ALGUES POUR AUGMENTER LA REPONSE IMMUNITAIRE CHEZ LES MAMMIFERES

(30) Priority: 21.12.1999 US 469176
(43) Date of publication of application: 05.03.2003
(73) Proprietor: TEXAS TECH UNIVERSITY, Lubbock, TX 79409-2007 (US)
(72) Inventor: ALLEN, Vivien Gore, Lubbock, TX 79407-2252 (US); POND, Kevin, R., Wolfforth, TX 79382 (US)
(74) Representative: Burford, Anthony Frederick
(86) International application number: PCT/US2000/016318
(87) International publication number: WO 2001/045724

(56) References cited:
- US-A- 5 229 118
- US-A- 5 276 056
- DATABASE WPI Section Ch, Week 199712 Derwent Publications Ltd., London, GB; Class B04, AN 1997-130915 XP002237108 & SU 1 642 725 A (AS USSR FAR E PACIFIC BIOORG CHEM INST), 10 August 1996 (1996-08-10)
- DATABASE WPI Section Ch, Week 197918 Derwent Publications Ltd., London, GB; Class C03, AN 1979-34537B XP002237109 & JP 54 040176 A (TONO-OKA K), 28 March 1979 (1979-03-28)
- DATABASE WPI Section Ch, Week 199815 Derwent Publications Ltd., London, GB; Class D13, AN 1998-167575 XP002237110 & RU 2 086 147 C (FITOLON CO LTD), 10 August 1997 (1997-08-10)
- ALLEN V G ET AL: "EFFECTS SEAWEED TREATMENT OF TALL FESCUE ON GRAZING STEERS" AMERICAN FORAGE AND GRASSLAND COUNCIL PROCEEDINGS, XX, XX, 1997, pages 168-172, XP002939125
- DATABASE BIOSIS [Online] CHARREAU ET AL.: 'Efficiency of fucans in protecting porcine endothelial cells against complement activation and lysis by human serum', XP002939672 Database accession no. 1997:190627 & TRANSPLANTATION PROCEEDINGS vol. 29, no. 1-2, 1997, pages 889 - 890
- DATABASE DRUGU [Online] KLINGER ET AL.: 'Anti-HIV activity of sulfated polysaccharides from the brown seaweed ascophyllum nodosum', XP002939673 Database accession no. 91-25081 & ANTIVIRAL RES. vol. 15, no. 69, SUPPL. 1, 1991,

## Description

The invention relates to mitigating the stress of weaning in lactating mares.

Seaweeds have been used through antiquity in crop production and, as early as the 1950's, evidence of plant growth hormones in seaweed was reported. Seaweed is now recognized as a source of plant growth regulators and has been demonstrated to have activity that includes cytokinin, auxin, gibberellin, and indole acetic acid. Seaweed has also long served as feed for domestic and wild animals. Some even graze on seaweed growing on rocky beaches and floating in the ocean water. Seaweeds have been dried and sold as a meal product to be mixed with other feed stuffs. The value of seaweed has been generally attributed to the fact that it is low in carbohydrate and proteins and rich in trace elements; including vitamins B, D, E and vitamin precursors including beta-carotene; and various growth hormones.

Morgan Herbs for Horses (The Kenilworth Press Limited, 1993) refers to the use of Kelp as a supplement for youngstock horses and for horses who are or have been on poor grazing. Reference is made to the possibility that internal use improves rheumatic and arthritic conditions

The present invention is directed to mitigating the stress in lactating mares, during and after weaning when the lactating mares are physically separated from their foals, by prior to weaning administering to the lactating mares a weaning stress mitigating effective amount of seaweed supplement. Lactating mares fed seaweed supplement prior to weaning had a substantially constant neutrophil to lymphocyte ratio for 40 days and longer after weaning whereas lactating mares in the control group (not fed seaweed supplement) had neutrophil to lymphocyte ratios that were more than double at 28 days after weaning.

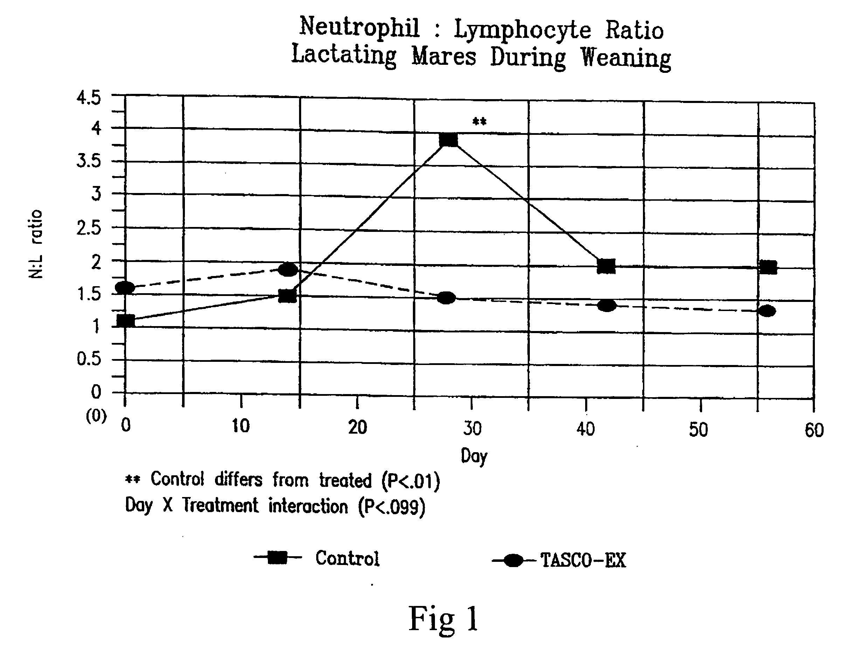

In the accompanying drawing:-
Figure 1 is a graphic presentation of neutrophil to lymphocyte ratio for lactating mares for 56 days after weaning for control diet and seaweed extract at 2% of total diet and shows results of Example II.

The seaweed supplement is, for example, seaweed extract or seaweed meal.

The seaweed from which the seaweed supplement is obtained can be from any of the various seaweed plant classifications, preferably those that have been utilized in agriculture and include seaweeds from the plant orders *Laminariaceae, Fucaceae* and *Gigartinaceae*. Genus groups include *Ascophyllum, Laminaria, Durvillea, Macrocystis, Chondrus* and *Ecklonia*. The seaweed for the preferred seaweed supplement herein is from the genus *Ascophyllum* which belongs to the order *Fucaceae* and is *Ascophyllum nodosum. Ascophyllum nodosum* is a brown seaweed which grows along the North Atlantic shorelines of Canada, the United States, and Europe.

We turn now to seaweed supplement which is seaweed extract.

Seaweed extract is water soluble and can be obtained by alkaline hydrolysis extraction. A preferred seaweed extract is obtained by alkaline hydrolysis extraction from *Ascophyllum nodosum*; commercial products of this kind are available from Acadian Seaplants Limited of Nova Scotia Canada, and are sold under the tradenames Acadian™ Soluble Seaweed Extract Powder (powder form), Acadian™ Liquid Seaweed Concentrate (liquid form), Tasco™-Ex (powder form) and Tasco™-Forage (powder form). Acadian™ Soluble Seaweed Extract Powder, Tasco™-Ex and Tasco™-Forage have the same composition. Acadian™ Soluble Seaweed Extract Powder is made up of brownish-black crystals, has a seaweed-like odour, is 100% soluble in water and has a pH of 10-10.5 in water and typical analysis shows by weight 6.5% maximum moisture, 45-55% organic matter, 45-55% ash (minerals), 1.0 - 2.0% total nitrogen (N), 2.0 - 4.0% available phosphoric acid (P₂O₅), 18.0 - 22.0% soluble potash (K₂O), 1.0 - 2.0% sulphur (S), 0.2 - 0.5% magnesium, 0.1 - 0.2% calcium, 3.0 - 5.0% sodium, 75 - 150 ppm boron, 75 - 250 ppm iron, 8 - 12 ppm manganese, 1 - 10 ppm copper, 25 - 75 ppm zinc; alginic acid, mannitol, and laminarin carbohydrates; cytokinin, auxin and gibberellin growth promoters; and the following average grams of amino acid per 100 grams of protein: alanine, 3.81; arginine, 0.22; aspartic acid, 5.44; cystine, trace; glutamic acid, 7.69; glycine, 3.16; histidine, 0.42; isoleucine, 1.94; leucine, 4.84; lysine, 1.33; methionine, 1.39; phenylalanine, 2.82; proline, 4.42; serine, 0.14; threonine, 1.27; tyrosine, 1.80, and valine, 3.46.

The seaweed extract can be applied, for example, in an amount ranging from 0.3 kg/ha to 5 kg/ha, e.g., 1 to 4 kg/ha, and an application amount of 3.4 kg/ha (3 lbs/acre) has been used with good advantage. The seaweed extract (powder form) is readily dissolved in 20 to 40 gallons of water per acre (185 to 375 litres/ha). Application is preferably carried out by spraying the water solution on the pasture forage using a commercial field-type of sprayer.

Seaweed extract is preferably admixed into diet for direct feeding by inclusion at the time of feeding by top dressing or mixing into the feed at the time of feeding or by premixing at the time the diet ingredients are combined and is included in an amount of, e.g., 0.01 to 3% by weight (powder or liquid concentrate commercial products) of the diet.

We turn now to seaweed supplement which is seaweed meal or flour.

The seaweed meal or flour can be obtained by dehydrating the seaweed, for example, by solar drying followed by low heat finish drying and processing the dehydrated material into a granular meal or flour. A preferred seaweed meal is obtained from *Ascophyllum nodosum* and is available from Acadian Seaplants Limited of Nova Scotia, Canada, and is sold under the tradenames Acadian™ Kelp Meal and Tasco™-14. Acadian™ Kelp Meal and Tasco™-14 have the same composition. A typical analysis of Acadian™ Kelp Meal shows the following approximate weight percentages: moisture 12.0%, crude protein 6.0%, crude fibre 6.0%, ash (minerals) 22.0%, fat 20%, and carbohydrates 52%. Analysis of Acadian™ Kelp Meal for carbohydrates gives by weight 18.0 - 27.0% alginic acid, 3.8 - 8.0% mannitol, 2.0 - 5.0% laminarin, and 20.0 - 22.0% other sugars. Analysis of Acadian Kelp Meal for minerals gives 50 - 150 ppm aluminium, 5 - 15 ppm barium, <1 ppm beryllium, 80 - 100 ppm boron, <1 ppm cadmium, 1.0 - 3.0% calcium, 1.0 - 3.0% chloride, 1 - 2 ppm chromium, < 1 ppm cobalt, 1 - 10 ppm copper, <1,000 ppm iodine, 100 - 500 ppm iron, <1 ppm lead, 0.5 - 1.0% magnesium, 10-50 ppm manganese, <1 ppm mercury, <2 ppm molybdenum, <1 ppm nickel, 0.5 - 2.0% nitrogen, 0.1 - 0.2% phosphorus, 1.5 - 2.5% potassium, 3 - 4 ppm selenium, 2.4 - 4.0% sodium, 100 - 600 ppm strontium, 2.0 - 3.0% sulphur, < 10 ppm tin, 1 - 10 ppm titanium, 2 - 6 ppm vanadium and 10 - 50 ppm zinc. Analysis of Acadian Kelp Meal for vitamins gives 0.1 - 0.4 ppm biotin, 30 - 60 ppm carotene, 0.1 - 0.5 ppm folic acid, 0.1 - 0.5 ppm folinic acid, 10 - 30 ppm niacin, 5 - 10 ppm riboflavin, 1 - 5 ppm thiamine, 150 - 300 ppm tocopherols, 100 - 2,000 ppm vitamin C,<0.004 ppm vitamin B₁₂, and <10 ppm vitamin K. Analysis of the amino acid content for Acadian Kelp Meal gave the following expressed as grams of amino acid per 100 g of protein nitrogen: alanine 5.3, arginine 8.0, aspartic acid 6.9, cystine (trace), glycine 5.0, glutamic acid 10.0, histidine 1.3, isoleucine 2.8, leucine 4.6, lysine 4.9, methionine 0.7, phenylalanine 2.3, proline 2.6, serine 3.0, threonine 2.8, tryptophan (trace), tyrosine 0.9, and valine 3.7.

Seaweed meal is preferably applied to a pasture to provide seaweed treated forage by application in dry form and solubles from seaweed meal dissolve after application so that the solubilized material is available for foliar uptake and/or leaches into the ground and is taken up by the forage. The seaweed meal can be applied, for example, in an amount of 0.3 to 10 kg per acre.

Seaweed meal is preferably admixed into diet for direct feeding by inclusion at the time the diet ingredients are mixed or by directed addition at the time of feeding, in an amount of, e.g., 0.01 to 5%, by weight of the diet.

Recently there has been a particular interest in how stress-related physiological changes impinge upon immune function. The end of lactation (milk production) has components of both a physiological and psychological stress. At weaning the young foal is removed from its mother which is still responding physiologically by producing milk. The mother may still be able to see and hear her foal, which, along with the physical separation creates anxiety in both the mother and foal. Such anxiety stresses the mares and affects the immune response of the mares by decreasing the number of lymphocytes resulting in increased neutrophil to lymphocyte ratio. This effect is caused by stress hormones. The principal stress hormone, cortisol, has been shown to cause a shift in the number of various white blood cells causing increase in neutrophil to lymphocyte ratio and decreasing immune response. This aspect of the invention stabilizes the neutrophil to lymphocyte ratio and prevents significant increase thereof in mares after weaning thereby mitigating the stress of weaning.

For mitigating the stress of weaning in lactating mares, feeding of the seaweed supplement is preferably started at least five days prior to weaning, very preferably at least 10 days prior to weaning or 10 to 30 days prior to weaning, e.g., starting 14 days prior to weaning, and is preferably continued until weaning occurs or even after weaning occurs, e.g., up to seven days after weaning occurs.

The seaweed supplement is directly fed to the mares in admixture with the diet or is applied to pasture forage consumed by the mares, as described above. The seaweed supplement is administered in a weaning stress mitigating effective amount as indicated by stabilization of neutrophil to lymphocyte ratio to an increase of less than 40% or even a decrease, e.g., of up to 20 or 30%. For direct feeding, admixture with the diet is readily carried out as described above and when the seaweed supplement is seaweed extract, it is fed, for example, in an amount, for example, of 0.01% to 3% by weight (powder or liquid concentrate forms of seaweed extract) of the diet and when the seaweed supplement is seaweed meal, it is fed, for example, in an amount of, for example, 0.01 to 5% by weight of the diet. The seaweeds described above in conjunction with the broadest form of the invention are useful to provide the seaweed supplement for this weaning stress mitigating embodiment, and preferably the seaweed supplement is from *Ascophyllum nodosum.*

The invention is illustrated by the following example.

### Example I

In this study, 10 mares and their foals were fed seaweed extract (Tasco™-Ex) in amount of 2% of the total diet and 10 were fed a normal diet, for 14 days prior to weaning.

The diet aside from seaweed supplement was as follows: 46% oats, 37% corn, 5.5% soybean meal, 5.4% molasses, 4% fat, 1.1% dicalcium phosphate, 0.7% calcium carbonate and 0.3% vitamin/mineral premix.
The seaweed extract powder was admixed with diet to provide diet for the mares fed seaweed supplemented diet by hand mixing into the diet at the time of feeding. Each mare was individually fed.

Blood samples were collected at the start of the study, at weaning, and three times more to determine the effect of weaning and handling on the neutrophil to lymphocyte ratio aspect of the immune system.

The results are shown in Fig. 1.

Normally, horses have about 54% neutrophils and 35% lymphocytes which is a ratio of neutrophils to lymphocytes of about 1.5:1. As indicated in Fig. 1, control mares (not fed seaweed supplement) had elevated neutrophil to lymphocyte ratio increasing to near 4.0 on day 28. As shown in Fig. 1, for mares fed diet with seaweed supplement, the neutrophil to lymphocyte ratio was consistent at around 1.5.

These findings indicate that feeding seaweed supplement to lactating mares prior to weaning mitigated the stress of weaning and handling, especially on day 28.

Similar results of mitigating the stress of weaning and handling are obtained, when the lactating mares are grazed on seaweed extract treated pasture or seaweed meal treated pasture instead of being directly feed seaweed extract and diet as described above.

## Claims

1. The use of seaweed supplement in the manufacture of a product for mitigating the stress of weaning in lactating mares during and after weaning when lactating mares are physically separated from their foals.

2. A use of Claim 1, wherein the product is for administration of seaweed supplement starting at least 10 days prior to weaning and continued until weaning.

3. A use of Claim 1 or Claim 2, wherein the product is for administration to the lactating mares by grazing on seaweed supplement treated pastures.

4. A use of any one of the preceding claims, wherein the seaweed supplement is seaweed extract obtained by alkaline hydrolysis extraction of seaweed.

5. A use of Claim 3, wherein the seaweed supplement is seaweed extract obtained by alkaline hydrolysis extraction of seaweed and is for administration to the pasture in an amount ranging from 0.3 kg/ha to 5 kg/ha.

6. A use of any one of Claims 1 to 3, wherein the seaweed supplement is seaweed meal obtained by dehydrating seaweed.

7. A use of Claim 3, wherein the seaweed supplement is seaweed meal obtained by dehydrating the seaweed and is for administration to the pasture in an amount of 0.3 to 10 kg per acre.

8. A use of any one of the preceding claims, wherein the seaweed supplement is from Ascophyllum nodosum.

9. A use of any one of Claims 1, 2, 4 or 6, wherein the product is administered by directly feeding seaweed supplement in admixture with diet.

10. A use of Claim 9, wherein the seaweed supplement is a powder or liquid concentrate form of a seaweed extract as defined in Claim 7 and the product is for administration to provide said form in an amount of 0.01 to 3% by weight of the diet.

11. A use of Claim 9, wherein the seaweed supplement is seaweed meal and the product is for administration to provide said meal in an amount of 0.1 to 5% by weight of the diet.

## Patentansprüche

1. Verwendung eines Meeresalgen-Zusatzes bei der Herstellung eines Produkts zur Linderung der Entwöhnungsbelastung bei laktierenden Stuten während und nach der Entwöhnung, wenn laktierende Stuten physisch von ihren Fohlen getrennt sind.

2. Verwendung nach Anspruch 1, bei der das Produkt zur Verabreichung eines Meeresalgen-Zusatzes, die mindestens 10 Tage vor der Entwöhnung beginnt und bis zur Entwöhnung fortgesetzt wird, ist.

3. Verwendung nach Anspruch 1 oder Anspruch 2, bei der das Produkt zur Verabreichung an die laktierenden Stuten durch weiden lassen auf Weideland, das mit einem Meeresalgen-Zusatz behandelt wurde, ist.

4. Verwendung nach irgendeinem der vorangehenden Ansprüche, bei der der Meeresalgen-Zusatz ein Meeresalgen-Extrakt ist, der durch alkalische Hydrolyse-Extraktion von Meeresalgen erhalten wurde.

5. Verwendung nach Anspruch 3, bei der der Meeresalgen-Zusatz ein Meeresalgen-Extrakt ist, der durch alkalische Hydrolyse-Extraktion von Meeresalgen erhalten wurde und zur Applikation auf das Weideland in einer Menge in dem Bereich von 0,3kg/ha bis 5 kg/ha ist.

6. Verwendung nach irgendeinem der Ansprüche 1 bis 3, bei der der Meeresalgen-Zusatz ein Meeresalgen-Mehl ist, das durch Dehydratisieren von Meeresalgen erhalten wurde.

7. Verwendung nach Anspruch 3, bei der der Meeresalgen-Zusatz ein Meeresalgen-Mehl ist, das durch Dehydratisieren der Meeresalgen erhalten wurde und zur Applikation auf das Weideland in einer Menge von 0,3 bis 10 kg pro Acre ist.

8. Verwendung nach irgendeinem der vorangehenden Ansprüche, bei der der Meeresalgen-Zusatz von Ascophyllum nodosum ist.

9. Verwendung nach irgendeinem der Ansprüche 1, 2, 4 oder 6, bei der das Produkt durch direktes Füttern des Meeresalgen-Zusatzes im Gemisch mit Nahrung verabreicht wird.

10. Verwendung nach Anspruch 9, bei der der Meeresalgen-Zusatz eine Pulverform oder eine flüssige Konzentratform eines Meeresalgen-Extrakts ist, wie in Anspruch 7 definiert, und bei der das Produkt zur Verabreichung ist, um die Form in einer Menge von 0,01 bis 3 Gew.% der Nahrung bereitzustellen.

11. Verwendung nach Anspruch 9, bei der der Meeresalgen-Zusatz Meeresalgen-Mehl ist, und bei der das Produkt zur Verabreichung ist, um das Mehl in einer Menge von 0,1 bis 5 Gew.% der Nahrung bereitzustellen.

## Revendications

1. Utilisation d'un complément d'algues dans la préparation d'un produit destiné à atténuer le stress de sevrage chez des juments allaitantes pendant et après le sevrage lorsque les juments allaitantes sont séparées physiquement de leurs poulains.

2. Utilisation selon la revendication 1, où le produit est destiné à l'administration d'un complément d'algues en commençant au moins 10 jours avant le sevrage et en poursuivant jusqu'au sevrage.

3. Utilisation selon la revendication 1 ou la revendication 2, où le produit est destiné à l'administration à des juments allaitantes par broutage de pâture traitée par le complément d'algues.

4. Utilisation selon l'une quelconque des revendications précédentes, où le complément d'algues est un extrait d'algues obtenu par extraction par hydrolyse alcaline d'algues.

5. Utilisation selon la revendication 3, où le complément d'algues est un extrait d'algues obtenu par extraction par hydrolyse alcaline d'algues et est destiné à l'administration à la pâture en une quantité allant de 0,3 kg/ha à 5 kg/ha.

6. Utilisation selon l'une quelconque des revendications 1 à 3, où le complément d'algues est une farine d'algues obtenue par déshydratation d'algues.

7. Utilisation selon la revendication 3, où le complément d'algues est une farine d'algues obtenue par déshydratation des algues et est destinée à l'administration à la pâture en une quantité de 0,3 à 10 kg/ha.

8. Utilisation selon l'une quelconque des revendications précédentes, où le complément d'algues provient de Ascophyllum modosum.

9. Utilisation selon l'une quelconque des revendications 1, 2, 4 ou 6, où le produit est administré par ingestion directe du complément d'algues en mélange avec le régime alimentaire.

10. Utilisation selon la revendication 9, où le complément d'algues est une forme concentrée en poudre ou liquide d'un extrait d'algues selon la revendication 7 et le produit est destiné à l'administration pour fournir ladite forme en une quantité de 0,01 à 3 % en poids du régime alimentaire.

11. Utilisation selon la revendication 9, où le complément d'algue est de la farine d'algues et le produit est destiné à l'administration pour fournir ladite farine en une quantité de 0,1 à 5 % en poids du régime alimentaire.
